# EUROPEAN PATENT APPLICATION

(11) **EP 2 287 157 A1**
(43) Date of publication of application: **23.02.2011**
(21) Application number: 09742184.6
(22) Date of filing: 05.05.2009
(51) Int. Cl.: C07D 307/33, C07C 69/604, C07D 303/48, C07C 69/60, C07C 391/00, C07C 69/40

(54) **METHOD FOR OBTAINING CINATRINS C3 AND C1**

(30) Priority: 06.05.2008 ES 200801305
(71) Applicant: Consejo Superior de Investigaciones Cientificas, 28006 Madrid (ES)
(72) Inventor: NOHEDA MARÍN, Pedro, E-28006 Madrid (ES); LOZANO GORDILLO, Luis Miguel, E-28050 Madrid (ES); MAROTO QUINTANA, Sergio, E-28006 Madrid (ES)
(74) Representative: ABG Patentes, S.L.
(86) International application number: PCT/ES2009/070139
(87) International publication number: WO 2009/135978

(57) **Abstract**

The present invention relates to a process for obtaining cinatrins C₁ and C₃ and derivatives thereof which comprises the hydroxylation of a compound of formula (III). The invention also relates to the intermediates of said synthesis and to their use for obtaining cinatrins C₁ and C₃ and derivatives thereof.

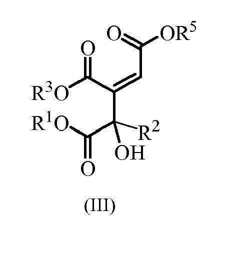

## Description

### Field of the Invention

The present invention relates to processes for the synthesis of cinatrins C₁ and C₃ and to intermediates of said synthesis. It also relates to the use of said intermediates in the synthesis of cinatrins C₁ and C₃.

### Background of the Invention

Cinatrins C₃ ((3*S*,4*S*,5*R*)-3,4-dihydroxy-3-dodecyl-4,5-dicarboxytetrahydro-2-furanone) and C₁ ((3*S*,4*S*,5*R*)-3,4-dihydroxy-5-dodecyl-4,5-dicarboxytetrahydro-2-furanone) belong to the family of Cinatrins.

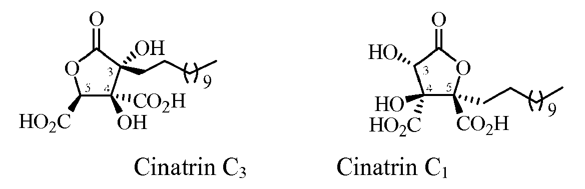

They were isolated in the year 1992 from the fungus *Circinotrichum falcatisporum* Pirozynsky (RF-641), isolated from living leaves of the India rubber tree *(Ficus elastica).* The structural elucidation of Cinatrins C3 and C1 was carried out by Dr. Itazaki's group in 1992 [Itazaki, H.; Nagashima, K.; Kawamura, Y.; Matsumoto, K.; Nakai, H.; Terui, Y. J. Antibiot. 1992, 45, 38-49.]. They also synthesized the dimethyl esters of cinatrins C₁ and C₃ from the natural compounds extracted from the fungus. However, the assignment of the absolute configuration performed by these authors is incorrect, and in the case of the dimethylated derivative of cinatrin C3 the following configuration was proposed:

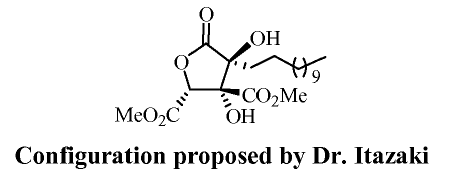

Said configuration was reviewed and corrected by Prof. Evans's group in 1997 [Evans, D. A.; Trotter, B. W.; Barrow, J. C. Tetrahedron 1997, 53, 8779-8794.], demonstrating that it corresponds to the enantiomer configuration proposed by Dr. Itazaki shown above.

Cinatrins C₁ and C₃ inhibit the action of the Phospholipase A₂ (PLA₂) enzyme and therefore have anti-inflammatory activity (Farooqui, A. A.; Litski, M. L.; Farooqui, T.; Horrocks, L. Brain Res. Bull. 1999, 49, 139-153; Piñón, P.; Kaski, J. C. Rev. Esp. Cardiol. 2006, 59, 247-258).

Two enantioselective syntheses of Cinatrins C₃ and C₁ have been described in the literature to date. Evans, D. A.; Trotter, B. W.; Barrow, J. C. Tetrahedron 1997, 53, 8779-8794 describes the first synthesis of these compounds. The key step in said synthesis consists of the stereoselective titanium-mediated aldol reaction of a tartrate-derived silylketene acetal, generated from (*R,R*)-tartaric acid di-*tert*-butyl ester, and an achiral α-keto ester, prepared from myristic acid benzyl ester. This synthesis is convergent, it comprises 5 steps and provides an overall yield of 24% for Cinatrin C₃ and 31% for Cinatrin C₁. However, the materials used are expensive and use various types of protecting groups.

Cuzzupe, A. N.; Di Florio, R.; White, J. M.; Rizzacasa, M. A. Org. Biomol. Chem. 2003, 1, 3572-3577 describes the second synthesis described to date for Cinatrins C₃ and C₁. The key step comprises the chelation-controlled addition of an organometallic reagent to an α-hydroxyketone in the presence of an ester, whereby the C4 quaternary stereogenic center of Cinatrin C₃ is generated. From the hydrolysis of the dimethyl ester of Cinatrin C₁ with sodium hydroxide, a mixture of Cinatrins C₃ and C₁ in a 1:1 ratio is obtained. The synthesis is linear, it comprises 18 steps and has an overall yield of 0.95%.

EP 0 405 864A2, US 5,120,647 and US 5,099,034 also describe obtaining cinatrins by means of isolation from the culture of *Circinotricum falcatisporum* RF-641, the subsequent hydrolysis thereof with sodium hydroxide to form the seco acid and esterification of the cinatrins to form their dimethyl esters by means of reaction with diazomethane.

It is therefore necessary to provide an improved synthesis of cinatrins C₁ and C₃ in terms of efficiency, yield and cost.

### Brief Description of the Drawings

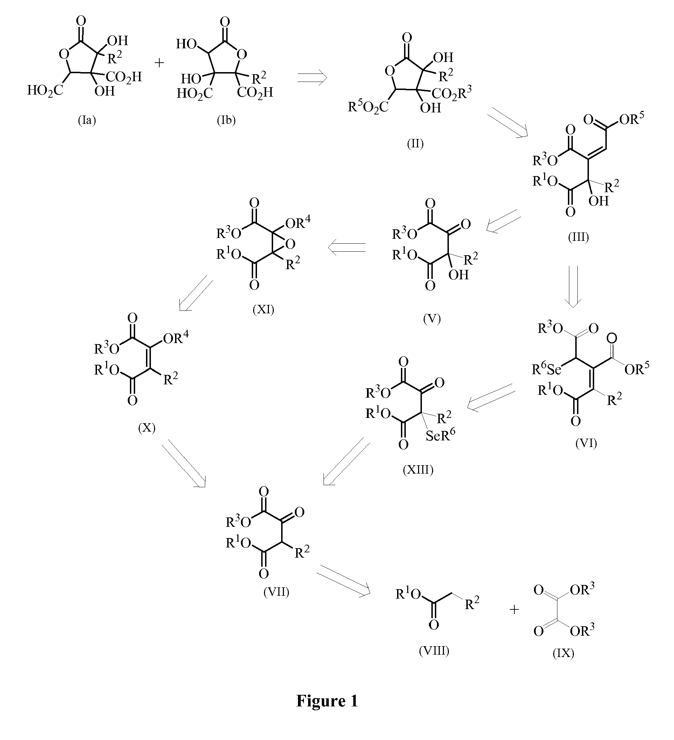

Figure 1 shows a retrosynthetic scheme of the sequence leading to the compounds of formula (Ia) and (Ib).

### Summary of the Invention

It has now been found that by following the synthetic sequence of the invention it is possible to obtain compounds of formula (Ia) and (Ib),including cinatrins C₁ and C₃, in a reduced number of steps and with a high yield. Said process has been made possible by means of using the new compounds of formula (II), (III), (V), (VI), (VII), (X), (XI) and (XIII).

Therefore, a first aspect of the present invention relates to a process for the synthesis of a compound of formula (II), an intermediate in the synthesis of the compounds of formula (Ia) and (Ib), including cinatrins C₁ and C₃, their stereoisomers, or mixtures thereof, from a compound of formula (III).

An additional aspect relates to the compounds of formula (III), an intermediate of the synthesis of the compounds of formula (Ia) and (Ib), their stereoisomers, or mixtures thereof, and to processes for obtaining them.

Other aspects of the present invention relate to compounds of formula (V), (VI), (VII), (X), (XI) and (XIII), intermediates in the synthesis of the compounds of formula (Ia) and (Ib), their stereoisomers, or mixtures thereof.

Another additional aspect relates to a process for obtaining the compounds of formula (Ia) and (Ib), their stereoisomers, or mixtures thereof, from a compound of formula (III).

Another additional aspect relates to a process for obtaining a compound of formula (Ia), its stereoisomers, or mixtures thereof, from a compound of formula (III).

Another additional aspect is aimed at a compound of formula (II) and to its use in the synthesis of the compounds of formula (Ia) or (Ib), their stereoisomers, or mixtures thereof.

An additional aspect relates to the use of at least one compound of formula (III), (V), (VI), (VII), (X), (XI) and (XIII) for the synthesis of cinatrins C₁ and C₃, their stereoisomers, especially enantiomers, or mixtures thereof, as well as mixtures of cinatrins C₁ and C₃ or mixtures of their stereoisomers, especially enantiomers.

### Detailed Description of the Invention

### Synthesis of cinatrins C₁ and C₃

According to a first aspect, the present invention is aimed at a process for obtaining a compound of formula (II), an immediate precursor of cinatrins C₁ and C₃, 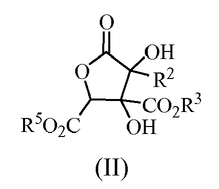 wherein
R² is a C₁₀-C₁₅ alkyl group; and
R³ and R⁵ are independently selected from a substituted or unsubstituted C₁-C₂₀ alkyl group;
its stereoisomers, or mixtures thereof;
which process comprises dihydroxylating the double bond of a compound of formula (III) 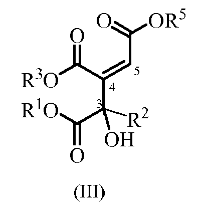 (the numbers next to each carbon indicate the numbering of the carbon atoms)
wherein
R², R³ and R⁵ are as defined above; and
R¹ is selected from a substituted or unsubstituted C₁-C₂₀ alkyl group.

Therefore, the process of the invention for obtaining the compounds of formula (II) first comprises a dihydroxylation of the carbon-carbon double bond of the compound of formula (III), followed by an intramolecular cyclization giving rise to the compound of formula (II). The stereochemistry of the dihydroxylation is directed by the hydroxyl in C3 of the compound of formula (III), therefore, depending on the stereochemistry in this carbon ((R) or (S)), different stereoisomers of the compounds of formula (Ia) and (Ib) can then be obtained. In other words, the stereoselective dihydroxylation and the subsequent cyclization are achieved in a single step. In any case, it is possible to invert the configuration of said hydroxyl by means of a Mitsunobu type reaction (for the specific case of inverting configuration of tertiary alcohols see Shi, Y. -J.; Hughes, D.L.; McNamara, J.M. Tetrahedron Lett. 2003, 44, 3609-3611; or Mukaiyama, T.; Shintou, T.; Fukumto, K. J. Am. Chem. Soc. 2003, 125, 10538-10539). Alternatively, if the compound of formula (III) is in racemic form, it will be possible to obtain the compounds of formula (Ia) and (Ib) in racemic form, being able to be used as such or separated into each of their enantiomers according to the methods which are common general knowledge.

According to a preferred embodiment, the compound of formula (III) is a compound of formula (IIIa), or its enantiomers 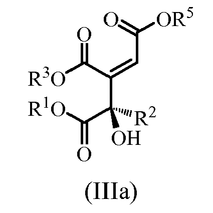 wherein R¹, R², R³ and R⁵ are as defined above;
and the compound of formula (II) obtained is a compound of formula (IIa), or its enantiomers 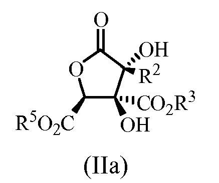 wherein R², R³ and R⁵ are as defined above.

The dihydroxylation reaction can be performed under conditions known by the skilled person, as described in Smith, M. B.; March, J. March's Advanced Organic Chemistry; John Wiley & Sons: New York, 2001. pp.: 1048-1051. According to a preferred embodiment, the dihydroxylation is performed in the presence of osmium tetroxide/*N*-methylmorpholine-*N*-oxide or potassium permanganate.

As observed in Figure 1, the preparation of the compound of formula (III) can be carried out by means of two alternative routes. Thus, according to a preferred embodiment, the compound of formula (III) is prepared by
(a) reacting a compound of formula (V) 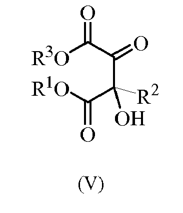 wherein
   R¹, R² and R³ are as defined above;
   in the presence of a compound of formula (XII) 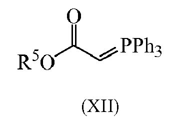 wherein
   R⁵ is as defined above;
   or
(b) oxidizing with a peroxide or with sodium periodate a compound of formula (VI) 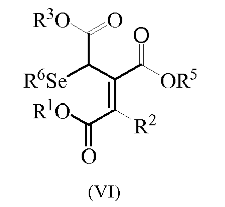 wherein
   R¹, R², R³ and R⁵ are as defined above; and
   R⁶ is selected from the group formed by C₁-C₃ alkyl and phenyl.

According to option a) the compound of formula (XII), a stabilized ylide, reacts with the compound of formula (V) to yield the compound of formula (III). Said stabilized ylide can be prepared according to known methods (Villa, M. J.; Warren, S. J. Chem. Soc. P. T 1 1994, 12, 1569-1572) or be commercially purchased. According to a preferred embodiment, said ylide is [(methoxycarbonyl)methylene]triphenylphosphorane. Following option b), the hydroxyl is introduced in C3 of the compound of formula (III) according to conditions known in the state of the art, for example with a peroxide, preferably hydrogen peroxide, or with sodium permanganate. Said introduction involves the sequence: (i) oxidation of the selenium atom, (ii) stereospecific 1,3-sigmatropic rearrangement, and (iii) release of the hydroxyl. For example, see Nishiyama, H.; Narimatsu, S.; Itoh, K. Tetrahedron Lett. 1981, 22, 5289-5292; or Werkhoven, T.M.; Nisper, R.; Lugtenburg, J. J. Eur. J. Org. Chem. 1999, 11, 2909-2914.

According to a preferred embodiment, and following synthetic route b), the compound of formula (VI) is prepared by reacting a compound of formula (XIII) 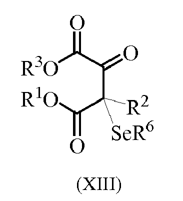 wherein
R¹, R², R³ and R⁶ are as defined above;
in the presence of a base and a phosphonate of formula (XIV) 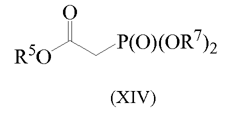 wherein
R⁵ is as defined above and R⁷ is a C₁-C₃ alkyl group.

According to a preferred embodiment, said base is selected from the group formed by sodium hydride, lithium di-*iso*-propylamine, 1,8-diazabicyclo[5.4.0]undec-7-ene (DBU) and 1,5-diazabicyclo[4.3.0]non-5-ene (DBN), preferably sodium hydride. The phosphonate of formula (XIV) used is preferably methyl (dimethoxyphosphoryl)acetate.

According to a preferred embodiment, the compound of formula (XIII) is prepared by reacting in the presence of a base a compound of formula (VII) 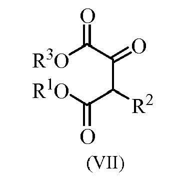 wherein
R¹, R² and R³ are as defined above;
with a compound of formula (XV)

R⁶SeX (XV)

wherein
R⁶ is as defined above, and
X is a halogen selected from C1 and Br.

Said base is preferably selected from the group formed by sodium hydride, a secondary amine such as morpholine, diethylamine, N-phthalimide or bis(trimethylsilyl)amides of alkali metals such as lithium (LiHMDS), sodium (NaHMDS) or potassium (KHMDS), preferably sodium hydride or morpholine.

Phenylselenyl bromide (PhSeBr) is preferably used as compound of formula (XV).

Said reaction can be carried out following methods known in the state of the art. For example, it is possible to add the compound of formula (XV) on a solution of sodium enolate generated from the compound of formula (VII) (see Smith, M. B.; March, J. March's Advanced Organic Chemistry; John Wiley&Sons: New York, 2001. pp.: 548-556.). Alternatively, it is possible to prepare a solution comprising an amine, for example morpholine, and the compound of formula (XV), and then add the compound of formula (VII) on said solution (see Boivin, S.; Outurquin, F.; Paulmier, C. Tetrahedron 1997, 53, 16767-16782.). According to a preferred embodiment, the base used is a chiral secondary amine, giving rise to an enantiomerically pure or enantiomerically enriched compound of formula (XIII). Therefore, known chiral secondary amines such as proline (see for example Vignola, N.; List, B. J. Am. Chem. Soc. 2004, 126, 450-451) allow obtaining the two enantiomers of the compounds of formula (XIII) or enantiomerically enriched mixtures thereof, and therefore the compounds of formula (VI), (III) and (II), and enantiomerically enriched or enantiomerically pure cinatrins C₁ and C₃.

Therefore, according to route b), the compounds of formula (VI) can be prepared following a synthetic route which comprises
(i) reacting a compound of formula (VII) with a compound of formula (XV) to obtain a compound of formula (XIII); and
(ii) reacting said compound of formula (XIII), in the presence of a base, with a phosphonate of formula (XIV).

Returning to route a) for obtaining the compounds of formula general (III), according to a preferred embodiment, the compound of formula (V) is prepared by reacting a compound capable of generating fluoride ions with a compound of formula (XI) 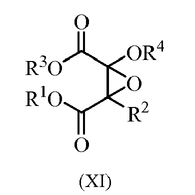 wherein
R¹, R² and R³ are as defined above, and
R⁴ is a trialkylsilyl group.

As can be observed, the epoxide group in the compound of formula (XI) opens regioselectively to form a compound of formula (V). General regioselective opening methods are known in the art, such as those described in Pujol, B.; Sabatier, R.; Driguez, P. A.; Doutheau, A. Tetrahedron Lett. 1992, 33, 1447-1450.

This opening is performed with a compound capable of generating fluoride ions. Preferably, the hydrofluoric acid-pyridine system, hydrofluoric acid in aqueous solution or a trihydrogen fluoride of formula NR₃·3HF, wherein R is independently selected from the group consisting of hydrogen and C₁-C₈ alkyl, is used; more preferably, the compound used is triethylamine tris-hydrofluoride (Et₃N·3HF).

Each of the two enantiomers of the compounds of formula (V) can be obtained by means of the regiospecific opening of the suitable enantiomer of the compound of formula (XI), when the latter is prepared by means of asymmetric epoxidation. Alternatively, if the compound of formula (XI) is in racemic form, the compound of formula (V) will also be obtained in its racemic form, being able to be used as such or separated into each of its enantiomers according to the methods which are common general knowledge.

According to a preferred embodiment, the compound of formula (XI) is obtained by reacting an epoxidizing agent with a compound of formula (X) 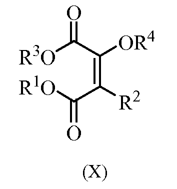 wherein
R¹, R², R³ and R⁴ are as defined above.

Non-limiting examples of conditions in which this transformation can be carried out can be found in, for example, a) Pujol, B.; Sabatier, R.; Driguez, P. A.; Doutheau, A. Tetrahedron Lett. 1992, 33, 1447-1450; or b) Lowinger, T. B.; Chu, J.; Spence, P. L. Tetrahedron Lett. 1995, 36, 8383-8386. It is also possible to find a general explanation about these reactions in the following references: (a) Smith, M. B.; March, J. March's Advanced Organic Chemistry; John Wiley & Sons: New York, 2001, pp. 1051-1054; (b) Davis, F. A.; Sheppard, A. C. J. Org. Chem. 1987, 52, 954-955; or (c) Dayan, S.; Bareket, Y.; Rozen, S. Tetrahedron 1999, 55, 3657-3664. According to a preferred embodiment, said epoxidizing agent is selected from the group consisting of *m-*CPBA, 2-sulfonyloxaziridines and the HOF·CH₃CN complex; more preferably *m*-CPBA.

As mentioned above, it is possible to perform the epoxidation of the compound of formula (X) in an asymmetric manner in order to obtain the compound of formula (XI) with an enantiomeric excess or in an enantiomerically pure manner, opening the route to compounds of formula (II), and therefore also to the compounds of formula (Ia) and (Ib), with an enantiomeric excess or in an enantiomerically pure manner.

Therefore, the present invention also contemplates the methods for the asymmetric epoxidation of the compounds of formula (X) such as those described in the art by means of using chiral auxiliaries as described in Walkup, R. D.; Obeyesekere, N. U. J. Org. Chem. 1988, 53, 920-923; or by means of using chiral catalysts as described in Zhu, Y.; Tu, Y.; Yu, H.; Shi, Y. Tetrahedron Lett. 1998, 39, 7819-7822.

According to a preferred embodiment, the compound of formula (X) is prepared by reacting a compound of formula (VII) 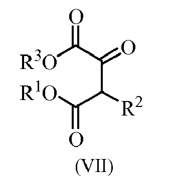 wherein
R¹, R² and R³ are as defined above;
with a trialkylsilyl halide or with a trialkylsilyl triflate in the presence of a base.

Non-limiting examples of conditions under which this transformation can be carried out can be found in, for example, Dalla, V.; Catteau, J. P. Tetrahedron 1999, 55, 6497-6510, and the trialkylsilyl groups which can be used in this reaction, as well as reagents suitable for their introduction, are known for the person skilled in the art (for example see Greene, T. W.; Wuts, P. G. M. Greene's Protective Groups in Organic Synthesis; John Wiley & Sons: Hoboken, 2007. pp.: 189-196.

According to a preferred embodiment, the trialkylsilyl group is selected from the group formed by trimethylsilyl, triethylsilyl, tri-iso-propylsilyl, dimethylisopropylsilyl, diethylisopropylsilyl, dimethylthexylsilyl, *tert*-butyldimethylsilyl and *tert-*butyldiphenylsilyl; and the preferred halides are selected from chlorine and iodine.

The trialkylsilyl group is preferably *tert*-butyldimethylsilyl; and the preferred trialkylsilyl triflate is *tert*-butyldimethylsilyl trifluoromethanesulfonate.

The reaction of the compounds of formula (VII) can give rise to two stereoisomers of the compounds of formula (X), according to the stereochemistry of the double bond in C2-C3 ((E) or (Z)). For the purposes of the present invention, it is irrelevant which of the two stereoisomers is formed. As has been mentioned above, it will be possible to invert the stereochemistry of the hydroxyl in the C3 position (for example, in compounds of (III) or (V)) by means of the Mitsunobu reaction, which allows obtaining any of the enantiomers of the compounds of formula (II), and therefore different stereoisomers of compounds of formula (Ia) and (Ib).

Therefore, according to route a), the compounds of formula (V) can be prepared following the synthetic route which comprises:
(i) reacting said compound of formula (VII) with a trialkylsilyl halide or with a trialkylsilyl triflate in the presence of a base to obtain a compound of formula (X);
(ii) reacting said compound of formula (X) with an epoxidizing agent to obtain a compound of formula (XI); and
(iii) reacting said compound of formula (XI) with a compound capable of generating fluoride ions.

As can be seen, in both routes a) and b), the common intermediate is a compound of formula (VII). According to a preferred embodiment, the compound of formula (VII) is prepared by reacting in the presence of a base a compound of formula (VIII) 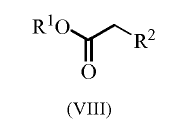 wherein
R¹ and R² are as defined above;
with an oxalic acid diester of formula (IX) 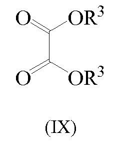 wherein
R³ is as defined above.

According to a preferred embodiment, said base is an inorganic base. Non-limiting examples of conditions under which this transformation can be carried out can be found in, for example, Dubowchik, G. M.; Padilla, L.; Edinger, K.; Firestone, R. A. J. Org. Chem. 1996, 61, 4676-4684. According to a preferred embodiment, said base is sodium hydride.

As mentioned above, the compounds of formula (Ia) and (Ib) can be prepared from the compounds of formula (III) by means of dihydroxylation followed by hydrolysis of the compound of formula (II) obtained in the presence of a base and subsequent acidification. Therefore, an additional aspect of the invention relates to a process for preparing compounds of formula (Ia) and (Ib), their stereoisomers, or mixtures thereof, or mixtures of the compounds of formula (Ia) and (Ib) or mixtures of their stereoisomers, which comprises
(i) dihydroxylating the double bond of a compound of formula (III) to obtain a compound of formula (II), its stereoisomers, or mixtures thereof;
(ii) hydrolyzing said compound of formula (II) in the presence of an alkali or alkaline earth metal hydroxide, or of an alkali or alkaline earth metal carbonate; and
(iii) acidifying the reaction medium;
wherein the R₃ and R₅ groups can be hydrolyzed under the basic conditions of step (ii).

As has been described throughout the present document, the synthesis of the compounds of formula (II), and therefore also of the compounds of formula (Ia) and (Ib), can be chiral by means of the synthesis of enantiomerically pure or enantiomerically enriched intermediates.

Therefore, a preferred embodiment of the present invention comprises the preparation of compounds of formula (Ia') and (Ib'), or their enantiomers 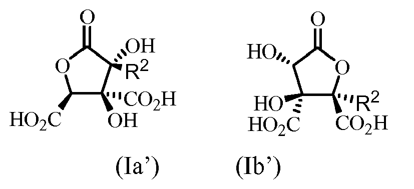 wherein
R² is as defined above;
from compounds of formula (IIIa) and (IIa), or their enantiomers.

As can be observed, the hydrolysis in basic medium and subsequent acidification (steps (ii) and (iii)) of the compounds of formula (II) involves the hydrolysis of the carboxy ester groups of which R₃ and R₅ form part to give rise to the corresponding carboxy acids. Therefore, in order to carry out the transformation indicated above it is necessary for the carboxy esters of which R₃ and R₅ form part to be labile in basic medium. Without wishing to be bound by theory, it seems that the step (ii) of the process for preparing compounds of formula (Ia) and/or (Ib) involves the formation of a triacid, the lactonization of the hydroxyl of the C2 position of which with the carboxy acid group of the C4 position (see route A in scheme 2) would generate a compound of formula (Ia). In addition, it seems that the acidic medium used in step (iii) must allow the formation of a tertiary carbocation, generated by means of the loss of the OH group of the C4 position of the triester provided by the acidic medium used (see route **B** in scheme 2). The subsequent cyclization of the carboxy acid group of the C2 position with said carbocation in the C4 position would generate a compound of formula (Ib).

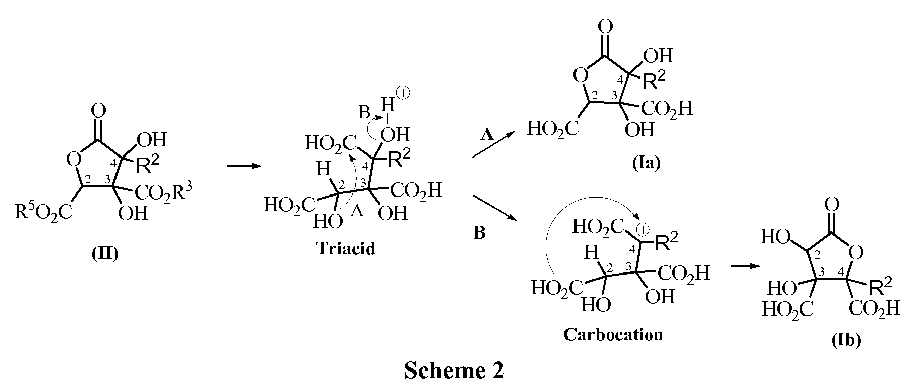

Suitable bases for the hydrolysis of carboxylic esters and for opening the ring in the compounds of formula (II) (step (i)) are known by the skilled person, such as lithium hydroxide, sodium hydroxide, potassium hydroxide, potassium carbonate, cesium carbonate, barium hydroxide.

Any protic acid will allow closing the cycle to form cinatrins C₁ and C₃ (step (ii)), the acid used is preferably hydrochloric acid.

Similar conditions for this reaction can be found in Cuzzupe, A. N.; Di Florio, R.; White, J. M.; Rizzacasa, M. A. Org. Biomol. Chem. 2003, 1, 3572-3577.

Said process can give rise to mixtures of the corresponding compounds of formula (Ia) and (Ib), which can be separated into the corresponding essentially pure compounds by means of methods which are common general knowledge (for example, chromatographic column or recrystallization).

Alternatively, it is possible to directly obtain the compounds of formula (Ia) from compounds of formula (II) and (III) without needing to form compounds of formula (Ib). This can be achieved by transforming the carboxy ester groups of which R₃ and R₅ form part into carboxy acid groups under conditions which do not give rise to the opening of the lactone and therefore under conditions which do not allow the formation of the aforementioned triacid (see Scheme 2), the origin of the formation of both compounds of formula (Ia) and of formula (Ib). Consequently, an additional aspect of the present invention is a process for preparing a compound of formula (Ia) 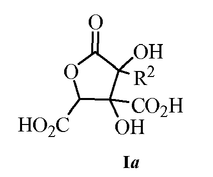 wherein R² is a C₁₀-C₁₅ alkyl group; its stereoisomers, or mixtures thereof, which comprises (i) dihydroxylating the double bond of a compound of formula (III), as defined above, to obtain a compound of formula (II) 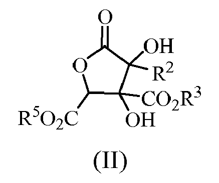 wherein R², R³ and R⁵ are as defined above; its stereoisomers, or mixtures thereof; and (ii) transforming under non-basic conditions the carboxy ester groups of the lactone ring of the compound of formula (II) to obtain the corresponding carboxy acid groups.

Conditions under which it is possible to perform the transformation of step (ii) are generally those in which it is possible to transform the carboxy ester groups of which R₃ and R₅ form part into carboxy acid groups under conditions which do not given rise to the opening of the lactone. Carboxy ester groups which can be transformed into carboxy acid groups under non-basic conditions (for example, by hydrogenation or in acidic medium) are known for the person skilled in the art. Non-limiting examples are esters derived from p-methoxybenzyl, 1-phenyl-ethyl, or trityl.

According to a preferred embodiment, R₃ and R₅ are benzyl groups (-(CH)₂-phenyl). The hydrogenation of a compound of formula (II) wherein R₃ and R₅ are benzyl only provides a compound of formula (Ia), without significant amounts of compounds of formula (Ib) being obtained. For similar conditions, see for example the transformation of compound 20 into compound 14 in Evans, D. A.; Trotter, B. W.; Barrow, J. C. Tetrahedron 1997, 53, 8779-8794, incorporated in its entirety to the description of the present invention. According to another preferred embodiment, it is possible for the R₃ and R₅ groups to be labile in acidic medium. The acidification of the reaction medium removes said R₃ and R₅ groups to form the corresponding carboxy acids, without opening the lactone of the compound of formula (II), therefore providing a compound of formula (Ia), without the formation of significant amounts of a compound of formula (Ib) being observed. For example, if R₃ and R₅ are t-butyl, it is possible to obtain the corresponding carboxy acids in acidic medium (for example, with trifluoroacetic acid) without the ring being opened. For example, see the transformation of compound 19 into compound 13 in Evans, D. A.; Trotter, B. W.; Barrow, J. C. Tetrahedron 1997, 53, 8779-8794.

According to a preferred embodiment, R² is n-dodecyl. According to another preferred embodiment, R¹ is a C₁-C₃ alkyl, preferably methyl. According to another preferred embodiment, R³ is a C₁-C₃ alkyl, preferably methyl. According to another preferred embodiment, R⁵ is a C₁-C₃ alkyl, preferably methyl.

### Intermediates of the process of the invention and use thereof

In view of what has been seen up until now, the compounds of formula (II) are intermediates in the synthesis of the present invention. Therefore, an additional aspect of the invention relates to a compound of formula (II) 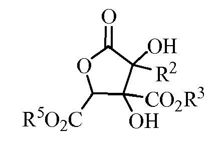 wherein
R² is selected from a C₁₀-C₁₅ alkyl group;
R³ and R⁵ are independently selected from a substituted or unsubstituted C₁-C₂₀ alkyl group;
its stereoisomers, especially enantiomers, or mixtures thereof;
with the proviso that the compound of formula (II) is not

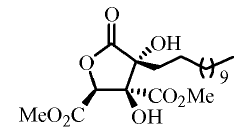

The use of the compounds of formula (II) defined above for the synthesis of compounds of formula (Ia) or (Ib), their stereoisomers, or mixtures thereof, as well as mixtures of compounds of formula (Ia) and (Ib) or mixtures of their stereoisomers, is also an aspect of the present invention.

Other aspects of the present invention relate to compounds of formula (III), (V), (VI), (VII), (X), (XI) and (XIII), intermediates in the synthesis of the invention, their stereoisomers, or mixtures thereof.

According to a preferred embodiment, R² in the compounds of formula (III), (V), (VI), (VII), (X), (XI) and (XIII) is n-dodecyl. According to another preferred embodiment, R¹ is methyl. According to another preferred embodiment, R³ is methyl. According to another preferred embodiment, R⁵ is methyl.

The use of the compounds of formula (III), (V), (VI), (VII), (X), (XI) and (XIII) for the synthesis of compounds of formula (Ia) or (Ib), their stereoisomers, or mixtures thereof, as well as mixtures of compounds of formula (Ia) and (Ib) or mixtures of their stereoisomers, is also an aspect of the present invention.

### Definitions

For the purpose of facilitating the understanding of the present invention, the meanings of several terms and expressions as they are used in the context of the invention are included herein.

"Alkyl" refers to a radical with a linear or branched hydrocarbon chain which consists of carbon and hydrogen atoms, which does not contain unsaturations and which is attached to the rest of the molecule by means of a single bond. The number of carbon atoms of the alkyl group is specified in each case. For example, when "C₁-C₄ alkyl" is indicated it refers to an alkyl group of one, two, three of four carbon atoms, i.e., methyl, ethyl, propyl, isopropyl or n-butyl. For example, when "C₁₀-C₁₅ alkyl" is indicated it refers to an alkyl group of ten, eleven, twelve, thirteen, fourteen or fifteen carbon atoms, such as decyl, undecyl, dodecyl, tridecyl, tetradecyl or pentadecyl.

"Halide" or "halogen" means-F, -Cl, -Br or -I;

A "stereoisomer" in the present application refers to compounds formed by the same atoms attached by the same sequence of bonds but having different three-dimensional structures which are not interchangeable.

"Enantiomer" is understood as the mirror image of a stereoisomerically pure compound. For the purposes of the invention, an enantiomer can be considered as a mixture of two enantiomers having an enantiomeric excess greater than 95%, preferably

greater than 98%, more preferably greater than 99%, more preferably greater than 99.5%.

"Bn" means benzyl (-(CH₂)-phenyl).

"Trialkylsilyl" is understood as a radical of formula -Si(R')(R")R"', wherein each of R', R" and R"' are independently selected from among a phenyl group and a C₁-C₆ alkyl group. Non-limiting examples of trialkylsilyl groups can be trimethylsilyl, triethylsilyl, tri-iso-propylsilyl; dimethylisopropylsilyl, diethylisopropylsilyl, dimethylthexylsilyl, *tert*-butyldimethylsilyl, *tert*-butyldiphenylsilyl.

The references of the present document to substituted groups in the compounds of the present invention refer to the specified moiety which can be substituted in one, two or three available positions with one, two, three suitable groups, which are independently selected from the group consisting of cyano; alkanoyl, such as a C₁-C₆ alkanoyl group, such as acyl and the like; carboxamido (-(C=O)NH₂); trialkylsilyl; carbocyclic aryl having 6 or more carbons, particularly phenyl or naphthyl and (C₁-C₃)alkylaryl such as tolyl. As a non-limiting example, "substituted alkyl" includes groups such as cyanoethyl, acetylmethyl, carboxamidomethyl (-CH₂CONH₂), 2-trimethylsilylethyl, benzyl, diphenylmethyl.

"Aryl" refers to a C₆-C₁₄ aromatic hydrocarbon radical such as phenyl, naphthyl or anthracyl.

Unless otherwise indicated, the compounds of the invention also refer to those including compounds which differ only in the presence of one or more isotopically enriched atoms. For example, the compounds having the present structures, with the exception of the substitution of a hydrogen with a deuterium or with tritium, or the substitution of a carbon with a ¹³C- or ¹⁴C-enriched carbon, are within the scope of this invention.

The following examples illustrate different embodiments of the invention and must not be considered as limiting the scope thereof.

### Examples

### General methods and materials

All the reactions were performed under an argon atmosphere, except those indicated in each case. The solvents used were distilled and dried under an argon atmosphere. The reagents and solvents used are from the companies Aldrich, Fluka, Merck, Sigma, Acros, Lancaster, SDS or Scharlau, and were purified by usual processes when necessary. The purification of the reaction products was performed by column chromatography under pressure (flash chromatography), using 60 Merck silica gel (with a 230-400 mesh particle size) as a stationary phase.

The (fully decoupled) ¹H and ¹³C nuclear magnetic resonance spectra were performed at room temperature in the solvent indicated in each case (CDCl₃ and CD₃OD) using the following apparatuses: Varian Gemini-200 (200 MHz), Varian INOVA-300 (300 MHz), Bruker Avance-300 (300 MHz) and Varian INOVA-400 (400 MHz). The values of the chemical shifts are expressed in parts per million (δ, ppm), using as an internal reference the residual signal of the solvent: CDCl₃, 7.26 ppm (¹H-NMR) and 77.0 ppm (¹³C-NMR); CD₃OD, 3.31 ppm (¹H-NMR) and 49.0 ppm (¹³C-NMR). The ¹H-NMR spectra are described indicating the number of protons and the apparent multiplicity of each signal. The coupling constants (*J*) are the apparent ones and are expressed in Hz. The following abbreviations have been used: s (singlet), d (doublet), t (triplet), c (quadruplet), q (quintuplet) and m (multiplet).

The melting points (m.p.) were measured in a Reichert brand Kofler microscope. The infrared (IR) spectra were recorded in the Perkin-Elmer spectrophotometer models 681 and FT-IR Spectrum One. The low resolution mass spectra (LRMS) were recorded: (1) by direct injection of the sample into a Hewlett Packard 5973 MSD spectrophotometer using the electron impact (EI) ionization technique; or (2) in a Hewlett Packard LCMS 1100 MSD spectrophotometer (an HPLC-coupled quadrupole analyzer) using the electrospray chemical ionization technique (API-ES) in positive or negative modes. The elemental analyses (E.A.) were performed with the Perkin-Elmer 240C and Heraus CHN-O-Rapid analyzers.

Unless otherwise indicated, all the products shown in the examples are racemic (*rac*).

### Example 1

### Preparation of methyl rac-(S)-3-(methoxycarbonyl)-2-oxopentadecanoate

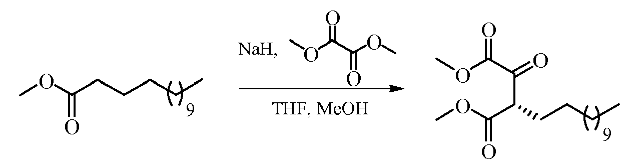

MeOH (0.5 ml) was added at 0°C to a suspension ofNaH (1.08 g, 45.3 mmoles) in THF (19.5 ml). The mixture was stirred until it reached room temperature. Then, methyl myristate (10 g, 41.2 mmoles) and dimethyl oxalate (4.87 g, 41.2 mmoles) were added. The resulting mixture was heated under reflux for 3 hours. After that time, H₂O (19 ml) was added at 0°C and the mixture was neutralized with an aqueous solution of 10% HCl. The phases were separated, and the aqueous phase was extracted with AcOEt (3×10 ml). The organic phase was dried with anhydrous Na₂SO₄, filtered and the solvent was removed under reduced pressure. The product was purified by a chromatographic column (hexane/AcOEt, 20:1), obtaining (8.61 g, yield 64%) methyl *rac*-(*S*)-3-(methoxycarbonyl)-2-oxopentadecanoate, as a transparent oil.
**¹H-NMR** (300 MHz, CDCl₃). δ 4.01 (1H, t, *J*= 6.9 Hz, H-3), 3.88 (3H, s, -OCH₃), 3.71 (3H, s, -OCH₃), 1.88 (2H, m, H-4), 1.24 (20H, m, -CH₂-), 0.86 (3H, m, -CH₃).

### Example 2

### Preparation of methyl 2-(tert-butyldimethylsilyloxy)-3-(methoxycarbonyl)-2-pentadecenoate

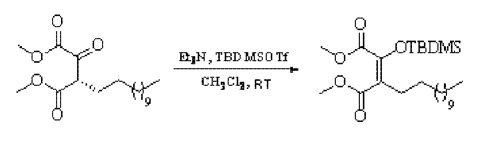

TBDMSOTf (1.84 g, 6.96 mmoles) was added at 0°C to a solution of methyl *rac*-(*S*)-3-(methoxycarbonyl)-2-oxopentadecanoate (1.90 g, 5.80 mmoles) and Et₃N (1.17 g, 11.6 mmoles) in CH₂Cl₂ (48 ml). The mixture was stirred at room temperature for 24 hours. After that time, the solvent was removed under reduced pressure. The product was purified by a chromatographic column (hexane/AcOEt, 12:1), obtaining (2.09 g, yield 82%) methyl 2-(*tert*-butyldimethylsilyloxy)-3-(methoxycarbonyl)-2-pentadecenoate, as a transparent oil.
**IR** (NaCl): v 3388, 2943, 2927, 2854, 1738, 1716, 1627, 1432, 1310, 1204, 1129, 1097, 840, 811, 784 cm⁻¹.
**¹H-NMR** (300 MHz, CDCl1₃). δ 3.79 (3H, s, -OCH₃), 3.71 (3H, s, -OCH₃), 2.29 (2H, m, -CH₂-), 1.32 (20H, m, -CH₂-), 0.94 (9H, s, *tert*-BuSi), 0.87 (3H, m, -CH₃), 0.17 (6H, s, (CH₃)₂Si).
¹³C-NMR (75 MHz, CDCl₃). δ 182.3, 168.6, 165.8, 118.2, 52.2, 51.7, 31.9, 29.6, 29.5, 29.4, 29.3, 28.2, 26.0, 25.4, 22.6, 18.3, 14.1, -4.6.
**LRMS(EI):** *m*/*z* 442(M⁺, 0), 441(M⁺-1, 1), 427(3), 411(3), 385(100).
**E.A.** (C₂₄H₄₆O₅Si): Found: C, 65.00, H, 10.50; Calculated: C, 65.11, H, 10.47.

### Example 3

### Preparation of methyl rac-(2R,3R)-2-(tert-butyldimethylsilyloxy)-2,3-epoxy-3-(methoxycarbonyl)-bentadecanoate

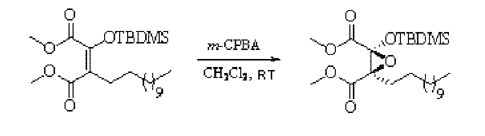

*m-*CPBA (7.77 g, 45.0 mmoles) was added to a solution of methyl 2-(*tert-*butyldimethylsilyloxy)-3-(methoxycarbonyl)-2-pentadecenoate (9.96 g, 22.5 mmoles) in CH₂Cl₂ (129 ml). The mixture was stirred at room temperature for 5 days. After that time, Celite was added and the solvent was removed under reduced pressure. The product was purified by a chromatographic column (hexane/AcOEt, 10:1), obtaining (9.96 g, yield 96%) methyl *rac*-(2*R*,3*R*)-2-(*tert*-butyldimethylsilyloxy)-2,3-epoxy-3-(methoxycarbonyl)-pentadecanoate, as a transparent oil.
**IR** (NaCl): v 3389, 2952, 2928, 2856, 1758, 1462, 1440, 1401, 1362, 1307, 1255, 1199, 1165, 1100, 1004, 843, 785 cm⁻¹.
**¹H-NMR** (300 MHz, CDCl₃). δ 3.76 (3H, s, -OCH₃), 3.75 (3H, s, -OCH₃), 2.07 (1H, m, H-4), 1.78 (1H, m, H-4), 1.46 (2H, m, H-5), 1.24 (18H, S_{broad}, -CH₂-), 0.91 (9H, s, *tert-*BuSi), 0.87 (3H, m, -CH₃), 0.20 (3H, s, (CH₃)₂Si), 0.15 (3H, s, (CH₃)₂Si).
**¹³C-NMR** (75 MHz, CDCl₃). δ 168.1, 166.4, 82.0, 68.0, 52.8, 52.4, 31.8, 29.5, 29.5, 29.4, 29.3, 29.3, 28.1, 25.3, 24.4, 22.6, 17.9, 14.0, -4.0, -4.6.
**LRMS(EI):** *m*/*z* 458(M⁺, 0), 443(2), 427(0), 401(66), 399(9), 341(10), 313(3), 281(2), 233(100).
**E.A.** (C₂₄H₄₆O₆Si): Found: C, 63.00, H, 10.00; Calculated: C, 62.84, H, 10.11.

### Example 4

### Preparation of methyl rac-(R)-3-hydroxy-3-(methoxycarbonyl)-2-oxopentadecanoate

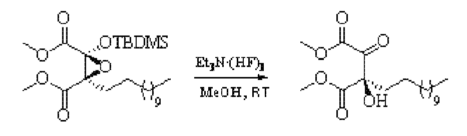

Et₃N·(HF)₃ (0.95 g, 5.90 mmoles) was added to a solution of methyl *rac-*(2*R*,3*R*)-2-(*tert*-butyldimethylsilyloxy)-2,3-epoxy-3-(methoxycarbonyl)pentadecanoate (9.01 g, 19.66 mmoles) in MeOH (180 ml). The mixture was stirred at room temperature for 2.5 hours. After that time, AcOEt was added, and the mixture was washed with H₂O (2 × 3 ml), dried with anhydrous Na₂SO₄, filtered and the solvent was removed under reduced pressure. The product, methyl *rac*-(*R*)-3-hydroxy-3-(methoxycarbonyl)-2-oxopentadecanoate, was used in the following step without purifying.
**IR** (NaCl): v 3473, 2955, 2925, 2854, 1740, 1438, 1259, 1138, 1074 cm⁻¹.
**¹H-NMR** (300 MHz, CDCl₃). δ 3.87 (3H, s, -OCH₃), 3.84 (3H, s, -OCH₃), 1.95 (2H, m, H-4), 1.24 (20H, S_{broad}, -CH₂-), 0.87 (3H, t, *J*= 6.5 Hz, -CH₃).
**¹³C-NMR** (75 MHz, CDCl₃). δ 188.5, 170.7, 162.0, 80.5, 53.6, 53.0, 33.9, 31.8, 29.5, 29.5, 29.5, 29.4, 29.2, 22.6, 22.4, 14.0.
**LRMS(EI):** *m*/*z* 345(M⁺+1,0), 313(1), 286(0), 257(14), 225(0), 197(100).

### Example 5

### Preparation of methyl rac-(2Z,4R)-4-hydroxy-3,4-bis(methoxycarbonyl)-2-hexadecenoate

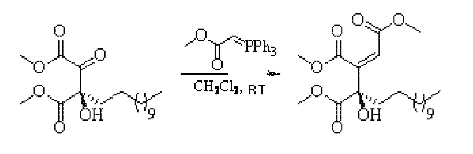

[(Methoxycarbonyl)methylene]triphenylphosphorane (16.43 g, 49.12 mmoles) was added to a solution of methyl *rac*-(*R*)-3-hydroxy-3-(methoxycarbonyl)-2-oxopentadecanoate (6.77 g, 19.65 mmoles), obtained previously, in CH₂Cl₂ (186 ml). The mixture was stirred at room temperature for 48 hours. After that time, Celite was added and the solvent was removed under reduced pressure. The product was purified by a chromatographic column (hexane/AcOEt, 7:1), obtaining (7.60 g, yield 97%) methyl *rac*-(2*Z*,4*R*)-4-hydroxy-3,4-bis(methoxycarbonyl)-2-hexadecenoate a s a colorless oil.
**IR** (NaCl): v 3494, 2925, 2854, 1733, 1646, 1436, 1349, 1256, 1199, 1168, 1019 cm⁻¹.
**¹H-NMR** (300 MHz, CDCl₃). δ 6.26 (1H, s, H-2), 3.80 (3H, s, -OCH₃), 3.80 (3H, s, - OCH₃), 3.71 (3H, s, -OCH₃), 3.67 (1H, s, -OH), 1.91 (2H, m, -CH₂-), 2.43 (2H, m, - CH₂-), 1.22 (18H, m, -CH₂-), 0.85 (3H, t, *J* = 6.8 Hz, -CH₃).
**¹³C-NMR** (75 MHz, CDCl₃). δ 173.0, 166.8, 164.9, 151.0, 120.3, 77.7, 53.6, 52.4, 52.0, 37.5, 31.8, 31.5, 29.5, 29.5, 29.4, 29.3, 29.3, 29.2, 23.1, 22.6, 14.0.
**LRMS(EI):** *m*/*z* 401(M⁺+1,3), 383(1), 369(8), 341(28), 309(100), 249(18), 197(18).
**E.A.** (C₂₁H₃₆O₇): Found: C, 63.00, H, 9.00; Calculated: C, 62.98, H, 9.06.

### Example 6

### Preparation of [(methoxycarbonyl)methylene]-triphenylphosphorane

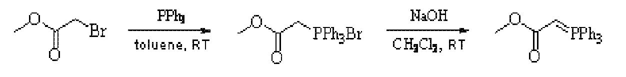

Methyl bromoacetate (5.3 g 34.6 mmoles, 1 eq.) was added to a solution of PPh₃ (9.52 g, 36.3 mmoles, 1.05 eq.) in toluene (20 ml). The mixture was stirred at room temperature for 24 hours. A suspension was gradually formed, which was filtered under vacuum and washed with toluene (3 × 10 ml). The product, [(methoxycarbonyl)methyl]triphenylphosphonium bromide, was used in the following step without purifying.
**¹H-NMR** (200MHz, CDCl₃). δ 8.00-7.60 (15H, m, Ar-H), 5.62 (1H, d, J_{P,H} = 15.5 Hz, H-1), 3.61 (3H, s, -OCH₃).

An aqueous solution of 1.15 N NaOH (60 ml) was added at room temperature to a solution of [(methoxycarbonyl)methyl]triphenylphosphonium bromide (14.3 g, 34.6 mmoles, 1 eq.), obtained previously, in CH₂Cl₂ (90 ml). The resulting mixture was stirred vigorously at room temperature for 2 hours. After that time, the phases were separated. The aqueous phase was extracted with CH₂Cl₂ (3 × 10 ml). The organic phase was dried with anhydrous MgSO₄, filtered and the solvent was removed under reduced pressure, obtaining (10.5 g, yield 90%) [(methoxycarbonyl)methylene]-triphenylphosphorane as a white solid.
**¹H-NMR** (200MHz, CDCl₃). δ 7.61 (15H, m, Ar-H), 5.62 (1H, m, H-1), 3.50 (3H, s, - OCH₃).

### Example 7

### Preparation of methyl rac-(R)-3-(phenylselenyl)-3-(methoxycarbonyl)-2-oxopentadecanoate

### Method A

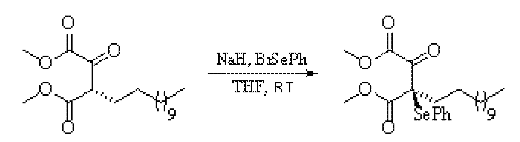

A solution of methyl *rac*-(*S*)-3-(methoxycarbonyl)-2-oxopentadecanoate (6 g, 18.26 mmoles) in THF (17 ml) was added to a suspension of NaH (0.482 g, 20.09 mmoles) in THF (34 ml). The mixture was stirred at room temperature until H₂ was no longer evolved (10 minutes). Then, the mixture was cooled at 0°C and a solution of BrSePh (4.74 g, 20.09 mmoles) in THF (17 ml) was added. The resulting mixture was stirred at room temperature for 24 hours. After that time, H₂O (50 ml) was added. The phases were separated, and the aqueous phase was extracted with AcOEt (3 × 30 ml). The organic phase was dried with anhydrous Na₂SO₄, filtered and the solvent was removed under reduced pressure. The product was purified by a chromatographic column (hexane/AcOEt, 10:1), obtaining (5.63 g, yield 65%) methyl *rac-(R)*-3-(phenylselenyl)-3-(methoxycarbonyl)-2-oxopentadecanoate, as a brown oil.

### Method B

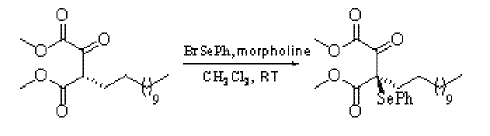

Morpholine (0.221 g, 2.54 mmoles) was slowly added at room temperature to a solution of BrSePh (0.300 g, 1.27 mmoles) in CH₂Cl₂ (12 ml). The resulting mixture was stirred at room temperature for 15 minutes. Then, a solution of methyl *rac-*(*S*)*-3-*(methoxycarbonyl)-2-oxopentadecanoate (0.417 g, 1.27 mmoles) in CH₂Cl₂ (2 ml) was added. The resulting mixture was stirred at room temperature for 24 hours, during which time a solid in suspension gradually appeared. After that time, the solid was filtered under vacuum over Celite, and the solvent was removed under reduced pressure. The product was purified by a chromatographic column (hexane/AcOEt, 10:1), obtaining (0.368 g, yield 60%) methyl *rac*-(*R*)-3-(phenylselenyl)-3-(methoxycarbonyl)-2-oxopentadecanoate, as a brown oil.
**IR** (NaCl): v 3057, 2925, 2854, 1736, 1713, 1577, 1438, 1299, 1232, 1129, 1064, 1020, 999, 743, 692 cm⁻¹.
**¹H-NMR** (200 MHz, CDCl₃). δ 7.36 (5H, m, Ar-H), 3.95 (3H, s, -OCH₃), 3.81 (3H, s, - OCH₃), 1.70 (2H, m, -CH₂-), 1.43-1.24 (20H, m, -CH₂-), 0.88 (3H, m, -CH₃).
**¹³C-NMR** (50 MHz, CDCl₃). δ 179.6, 168.3, 161.2, 137.8, 130.1, 129.3, 129.0, 124.6, 64.9, 53.3, 53.2, 31.6, 30.7, 29.2, 29.0, 27.0, 25.4, 24.6, 24.3, 22.5, 22.2, 20.8, 14.0.
**LRMS(EI)*****:** m*/*z* 484(M⁺+1,96), 426(3), 397(100), 365(60), 337(50), 269(14), 235(12), 157(55).
**E.A.** (C₂₄H₃₆O₅Se): Found: C, 59.70, H, 7.30; Calculated: C, 59.62, H, 7.50.

### Example 8

### Reaction of methyl rac-(R)-3-(phenylselenyl)-3-(methoxycarbonyl)-2-oxopentadecanoate with methyl (dimethoxyphosphoryl)acetate

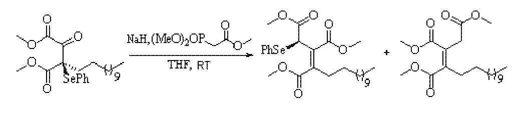

A solution of methyl (dimethoxyphosphoryl)acetate (2.33 g, 12.8 mmoles) in THF (8 ml) was added to a suspension ofNaH (0.307 g, 12.8 mmoles) in THF (91 ml). The mixture was stirred at room temperature until H₂ was no longer evolved (10 minutes). Then, a solution of methyl *rac*-(*R*)-3-(phenylselenyl)-3-(methoxycarbonyl)-2-oxopentadecanoate (5.63 g, 11.64 mmoles) in THF (8 ml) was added. The resulting mixture was stirred at room temperature for 24 hours. After that time, the solvent was removed under reduced pressure. The product was purified by a chromatographic column (hexane/AcOEt, 15:1), obtaining (4.53 g, yield 72%) a mixture in a 5:2 ratio of methyl *rac*-(*E,R*)-2-(phenylselenyl)-3,4-bis(methoxycarbonyl)-3-hexadecenoate *(**trans**,* 51%) and methyl (Z)-3,4-bis(methoxycarbonyl)-3-hexadecenoate (***cis**,* 21%), as a brown oil.
**IR** (NaCl): v 2925, 2854, 1733, 1632, 1577, 1458, 1435, 1313, 1268, 1199, 1158, 1125, 1075, 1001, 742, 693 cm⁻¹.
**¹H-NMR** (200 MHz, CDCl₃). δ 7.39 (5H, m, Ar-H ***trans),*** 4.64 (1H, s, H-2 ***trans),*** 3.71 (3H, s, -OCH₃ ***cis),*** 3.69 (3H, s, -OCH₃ ***trans**),* 3.68 (3H, s, -OCH₃ ***trans),*** 3.67 (3H, s, - OCH₃ ***trans),*** 3.65 (3H, s, -OCH₃ ***cis),*** 3.61 (3H, s, -OCH₃ ***cis**),* 3.33 (2H, s, H-2 ***cis),*** 2.23 (2H, m, H-5 ***cis**),* 1.83 (2H, m, H-5 ***trans),*** 1.13 (20H, m, -CH₂- ***trans, cis),*** 1.17 (20H, m, -CH₂- ***trans, cis*),** 0.79 (3H, m, -CH₃ ***trans),*** 0.76 (3H, m, -CH₃ ***cis).***
**¹³C-NMR** (50 MHz, CDCl₃). δ 169.9, 169.0, 166.2, 146.1, 143.1, 136.1, 129.8, 129.0, 128.8, 125.2, 52.9, 52.2, 52.1, 43.2, 33.5, 31.7, 31.2, 30.4, 29.4, 29.4, 29.3, 29.1, 29.1, 29.0, 27.3, 22.5, 20.8, 14.0, 13.9.
**LRMS(EI):** *m*/*z* 540(M⁺+1,6), 508(33), 383(12), 351(100), 291(8), 197(25).

### Example 9

### Preparation of methyl rac-(Z,R)-4-hydroxy-3,4-bis(methoxycarbonyl)-2-hexadecenoate

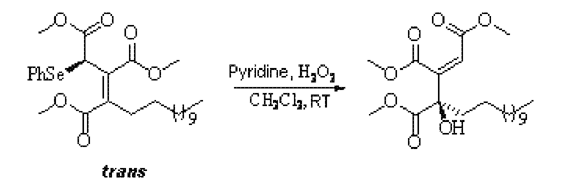

An aqueous solution of 33% H₂O₂ (5.99 ml) was added to a solution of methyl *rac*-(*E,R*)-2-(phenylselenyl)-3,4-bis(methoxycarbonyl)-3-hexadecenoate (***trans***) (4.53 g, 8.39 mmoles) and pyridine (1.32 g, 16.79 mmoles) in CH₂Cl₂ (70 ml). The mixture was stirred at room temperature for 24 hours. After that time, the solvent was removed under reduced pressure. The residue was dissolved in AcOEt (30 ml), and H₂O (30 ml) was added. The phases were separated, and the aqueous phase was extracted with AcOEt (3 × 10 ml). The organic phase was dried with anhydrous Na₂SO₄, filtered and the solvent was removed under reduced pressure. The product was purified by a chromatographic column (hexane/AcOEt, 10:1), obtaining (2.2 g, yield 65%) methyl *rac-*(*Z,R*)-4-hydroxy-3,4-bis(methoxycarbonyl)-2-hexadecenoate as a colorless oil.
**IR** (NaCl): v 3494, 2925, 2854, 1733, 1646, 1436, 1349, 1256, 1199, 1168, 1019 cm⁻¹.
**¹H-NMR** (300 MHz, CDCl₃). δ 6.26 (1H, s, H-2), 3.80 (3H, s, -OCH₃), 3.80 (3H, s, - OCH₃), 3.71 (3H, s, -OCH₃), 3.67 (1H, s, -OH), 1.91 (2H, m, -CH₂-), 2.43 (2H, m, - CH₂-), 1.22 (18H, m, -CH₂-), 0.85 (3H, t, *J* = 6.8 Hz, -CH₃).
**¹³C-NMR** (75 MHz, CDCl₃). δ 173.0, 166.8, 164.9, 151.0, 120.3, 77.7, 53.6, 52.4, 52.0, 37.5, 31.8, 31.5, 29.5, 29.5, 29.4, 29.3, 29.3, 29.2, 23.1, 22.6, 14.0.
**LRMS(EI):** *m*/*z* 401(M⁺+1,3), 383(1), 369(8), 341(28), 309(100), 249(18), 197(18).
**E.A.** (C₂₁H₃₆O₇): Found: C, 63.00, H, 9.00; Calculated: C, 62.98, H, 9.06.

### Example 10

### Preparation of rac-(3R,4S,5S)-3,4-dihydroxy-3-dodecyl-4,5-bis(methoxycarbonyl)tetrahydro-2-furanone (IV)

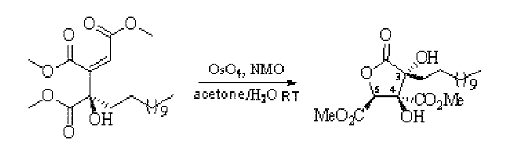

NMO (1.21 g, 9.99 mmoles) and OsO₄ (2.5% in *tert*-BuOH, 0.019 g, 0.075 mmoles) were added to a solution of methyl *rac*-(*Z,R*)-4-hydroxy-3,4-bis(methoxycarbonyl)-2-hexadecenoate (1 g, 2.50 mmoles) in a 5:1 acetone/H₂O mixture (10.2 ml). The mixture was stirred at room temperature for 2 days. After that time, an aqueous solution of 10% Na₂S₂O₃ (0.2 ml) was added. The mixture was filtered through silica gel with MeOH and the solvent was removed under reduced pressure. The product was purified by a chromatographic column (hexane/AcOEt, 3:1), obtaining (0.670 g, yield 67%) *rac*-(3*R*,4*S*,5*S*)-3,4-dihydroxy-3-dodecyl-4,5-bis(methoxycarbonyl)tetrahydro-2-furanone (**IV**) as a white solid.
**m.p.:** 74-76°C
**IR** (KBr): v 3400, 3339, 2956, 2918, 2851, 1785, 1775, 1714, 1467, 1445, 1364, 1307, 1226, 1155, 1133, 1061 cm⁻¹.
**¹H-NMR** (200 MHz, CDCl₃). δ 5.21 (1H, s, H-5), 3.87 (3H, s, -OCH₃), 3.78 (3H, s, - OCH₃), 3.56 (1H, S_{broad}, -OH), 2.90 (1H, S_{broad}, -OH), 1.80 (2H, m, H-6), 1.21 (20H, S_{broad}, -CH₂-), 0.83 (3H, m, -CH₃).
**¹H-NMR** (300 MHz, DMSO-*d₆*). δ 6.74 (1H, s, -OH), 6.41 (1H, s, -OH), 5.50 (1H, s, H-5), 3.72 (3H, s, -OCH₃), 3.65 (3H, s, -OCH₃), 1.68 (2H, m, H-6), 1.39 (2H, m, H-7), 1.23 (18H, S_{broad}, -CH₂-), 0.84 (3H, m, -CH₃).
**¹³C-NMR** (50 MHz, CDCl₃). δ 173.9, 170.6, 166.4, 81.0, 78.5, 54.0, 52.7, 31.7, 29.6, 29.5, 29.4, 29.2, 29.2, 22.5, 21.5, 13.9.
**LRMS(EI):** *m*/*z* 402(M⁺, 0), 315(2), 285(2), 234(2), 216(0), 197(11), 160(30), 101 (100).
**E.A.** (C₂₀H₃₄O₈): Found: C, 59.70, H, 8.50; Calculated: C, 59.68, H, 8.51.

### Example 11

### Reaction of rac-(3R,4S,5S)-3,4-dihydroxy-3-dodecyl-4,5-bis(methoxycarbonyl)tetrahydro-2-furanone with LiOH/HCl

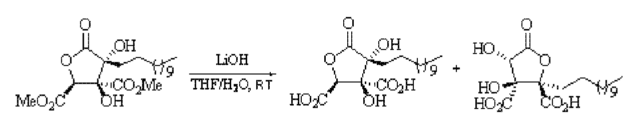

An aqueous solution of 1N LiOH (0.104 g, 2.48 mmoles, 2.5 ml) was added to a solution of *rac*-(3*R*,4*S*,5*S*)-3,4-dihydroxy-3-dodecyl-4,5-bis(methoxycarbonyl)tetrahydro-2-furanone **(IV)** (0.050 g, 0.12 mmoles) in THF (5 ml). The mixture was stirred at room temperature for 2 hours. After that time, the phases were separated. The aqueous phase was washed with AcOEt (2 × 2 ml), treated with an aqueous solution of 10% HCl (until acidic pH) and extracted with AcOEt (3 × 3 ml). The extracts were dried with anhydrous Na₂SO₄, filtered and the solvent was removed under reduced pressure, obtaining (0.026 g, yield 56%) a mixture in a 1:1 ratio of *rac-*(3*R*,4*S*,5*S*)-3,4-dihydroxy-3-dodecyl-4,5-dicarboxytetrahydro-2-furanone (Cinatrin C₃) and *rac*-(3*S*,4*S*,5*R*)-3,4-dihydroxy-5-dodecyl-4,5-dicarboxytetrahydro-2-furanone (Cinatrin C₁) as a white solid.
**¹H-NMR** (300 MHz, CD₃OD). δ 5.39 (1H, s, H-5, **Cinatrin** C₃), 4.70 (1H, s, H-3 **Cinatrin** C₁), 2.16 (1H, m, H-6 **Cinatrin** C₁), 1.82 (2H, m, H-6 **Cinatrin** C₃), 1.68 (1H, m, H-6 **Cinatrin** C₁), 1.50 (1H, m, H-7 **Cinatrin** C₁), 1.46 (2H, m, H-7 **Cinatrin** C₃), 1.36 (1H, m, H-7 **Cinatrin** C₁), 1.29 (18H, S_{broad}, -CH₂- **Cinatrin** C₃, **Cinatrin** C₁), 0.88 (3H, m, -CH₃ **Cinatrin C₃, Cinatrin** C₁).
**¹³C-NMR** (75 MHz, CD₃OD). **Cinatrin** C₃: δ 176.8, 172.6, 170.3, 82.1, 81.2, 80.8, 33.2, 32.1, 31.4, 30.8, 30.7, 30.5, 30.4, 23.6, 22.6, 14.5; **Cinatrin C₁:** δ 175.8, 172.0, 88.6, 85.7, 74.8, 33.4, 32.3, 31.6, 30.5, 30.4, 30.3, 30.1, 23.8, 22.4, 14.3.
**LRMS(EI):** *m*/*z* 374(M⁺, 0), 355(1), 331(1), 297(3), 267(3), 249(3), 207(5), 197(93), 57(100).

## Claims

1. A process for obtaining a compound of formula (II) 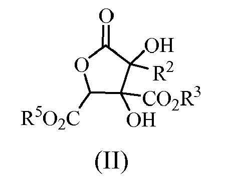 wherein
R² is aC₁₀-C₁₅ alkyl group; and
R³ and R⁵ are independently selected from a substituted or unsubstituted C₁-C₂₀ alkyl group;
its stereoisomers, or mixtures thereof;
which process comprises dihydroxylating the double bond of a compound of formula (III) 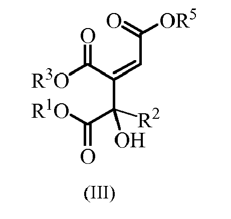 wherein
R², R³ and R⁵ have the previously mentioned meaning; and
R¹ is selected from a substituted or unsubstituted C₁-C₂₀ alkyl group.

2. The process according to claim 1, wherein the compound of formula (III) is a compound of formula (IIIa), or its enantiomers 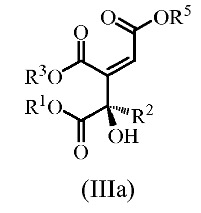 wherein R¹, R², R³ and R⁵ are as defined in claim 1;
and the compound of formula (II) obtained is a compound of formula (IIa), or its enantiomers 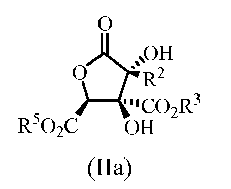 wherein R², R³ and R⁵ are as defined in claim 1.

3. The process according to any of the previous claims, wherein the dihydroxylation is performed in the presence of osmium tetroxide/*N*-methylmorpholine-N-oxide or potassium permanganate.

4. A compound of formula (III) 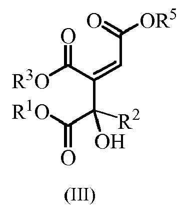 wherein
R¹, R³ and R⁵ are independently selected from a substituted or unsubstituted C₁-C₂₀ alkyl group; and
R² is selected from a C₁₀-C₁₅ alkyl group;
its stereoisomers, or mixtures thereof.

5. A process for the synthesis of a compound of formula (III) 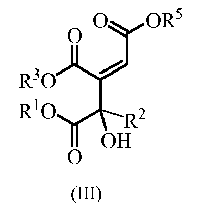 wherein
R¹, R³ and R⁵ are independently selected from a substituted or unsubstituted C₁-C₂₀ alkyl group; and
R² is a C₁₀-C₁₅ alkyl group;
its stereoisomers, or mixtures thereof;
which comprises
(a) reacting a compound of formula (V) 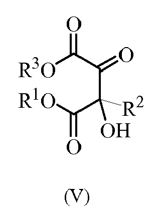 wherein
R¹, R² and R³ are as defined above;
in the presence of a compound of formula (XII) 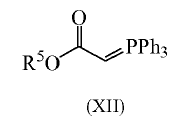 wherein
R⁵ is as defined above;
or
(b) oxidizing with a peroxide or with sodium periodate a compound of formula (VI) 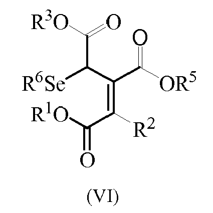 wherein
R¹, R², R³ and R⁵ are as defined above; and
R⁶ is selected from the group formed by C₁-C₃ alkyl and phenyl.

6. The process according to claim 5, wherein the compound of formula (VI) is prepared by
(i) reacting in the presence of a base a compound of formula (VII) 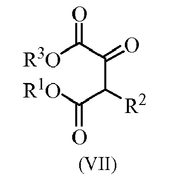 wherein
R¹, R² and R³ are defined as in claim 5;
with a compound of formula (XV)
R⁶SeX (XV)
wherein
R⁶ is defined as in claim 5; and
X is a halogen selected from Cl and Br;
to obtain a compound of formula (XIII) 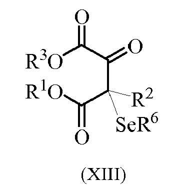 wherein
R¹, R², R³ and R⁶ are defined as in claim 5; and
(ii) reacting said compound of formula (XIII) in the presence of a base with a phosphonate of formula (XIV) 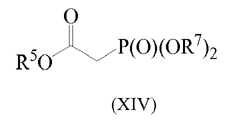 wherein
R⁵ is defined as in claim 5; and
R⁷ is a C₁-C₃ alkyl group.

7. The process according to claim 5, wherein the compound of formula (V) is prepared by
(i) reacting a compound of formula (VII) 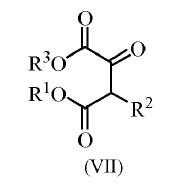 wherein
R¹, R² and R³ are defined as in claim 5;
with a trialkylsilyl halide or with a trialkylsilyl triflate in the presence of a base to obtain a compound of formula (X) 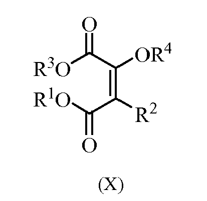 wherein
R¹, R² and R³ are defined as in claim 5; and
R⁴ is a trialkylsilyl group;
(ii) reacting said compound of formula (X) with an epoxidizing agent to obtain a compound of formula (XI) 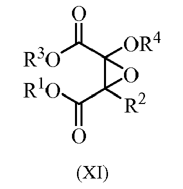 wherein
R¹, R² and R³ are defined as in claim 5; and
R⁴ is as defined above; and
(iii) reacting said compound of formula (XI) with a compound capable of generating fluoride ions.

8. The process according to claim 7, wherein said epoxidizing agent is selected from the group formed by 3-chloroperoxybenzoic acid, 2-sulfonyloxaziridines and the HOF·CH₃CN complex.

9. The process according to claim 7, wherein the epoxidation is asymmetric.

10. The process according to any of claims 6-9, wherein the compound of formula (VII) is prepared by reacting in the presence of a base a compound of formula (VIII) 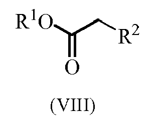 wherein
R¹ and R² are defined as in claim 6;
with an oxalic acid diester of formula (IX) 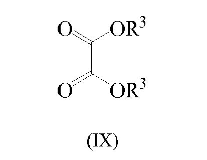 wherein
R³ is defined as in claim 6.

11. A compound of formula (V) 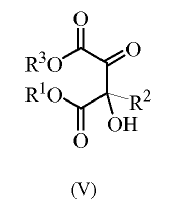 wherein
R¹ and R³ are independently selected from a substituted or unsubstituted C₁-C₂₀ alkyl group; and
R² is selected from a C₁₀-C₁₅ alkyl group;
its stereoisomers, or mixtures thereof.

12. A compound of formula (XI) 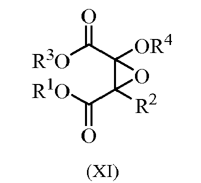 wherein
R¹ and R³ are independently selected from a substituted or unsubstituted C₁-C₂₀ alkyl group;
R² is selected from a C₁₀-C₁₅ alkyl group; and
R⁴ is a trialkylsilyl group;
its stereoisomers, or mixtures thereof.

13. A compound of formula (X) 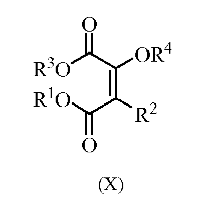 wherein
R¹ and R³ are independently selected from a substituted or unsubstituted C₁-C₂₀ alkyl group;
R² is selected from a C₁₀-C₁₅ alkyl group; and
R⁴ is a trialkylsilyl group
its stereoisomers or mixtures thereof.

14. A compound of formula (VI) 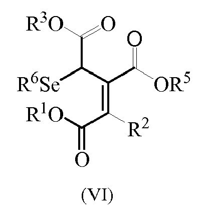 wherein
R¹, R³ and R⁵ are independently selected from a substituted or unsubstituted C₁-C₂₀ alkyl group;
R² is selected from a C₁₀-C₁₅ alkyl group; and
R⁶ is selected from the group formed by C₁-C₃ alkyl and phenyl;
its stereoisomers, or mixtures thereof.

15. A compound of formula (XIII) 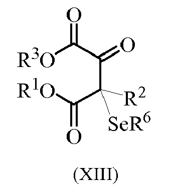 wherein
R¹ and R³ are independently selected from a substituted or unsubstituted C₁-C₂₀ alkyl group;
R² is selected from a C₁₀-C₁₅ alkyl group; and
R⁶ is selected from the group formed by C₁-C₃ alkyl and phenyl;
its stereoisomers, or mixtures thereof.

16. A compound of formula (VII) 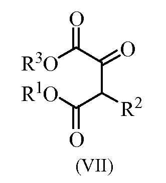 wherein
R¹ and R³ are independently selected from a substituted or unsubstituted C₁-C₂₀ alkyl group; and
R² is selected from a C₁₀-C₁₅ alkyl group;
its stereoisomers, or mixtures thereof.

17. The compound according to any one of claims 11 to 16, wherein R¹ = R³ = Me and R² = n-dodecyl.

18. A process for preparing compounds of formula (Ia) and/or (Ib) 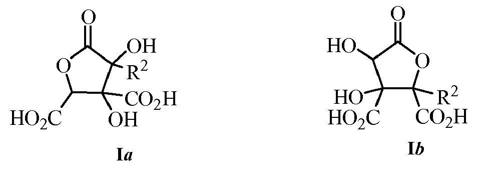 wherein
R² is a C₁₀-C₁₅ alkyl group;
their stereoisomers, or mixtures thereof, or mixtures of the compounds of formula (Ia) and (Ib) or mixtures of their stereoisomers,
which comprises
(i) dihydroxylating the double bond of a compound of formula (III) 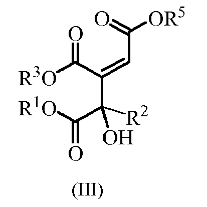 wherein
R¹ is selected from a substituted or unsubstituted C₁-C₂₀ alkyl group;
R² is as defined above; and
R³ and R⁵ are independently selected from a substituted or unsubstituted C₁-C₂₀ alkyl group;
to obtain a compound of formula (II) 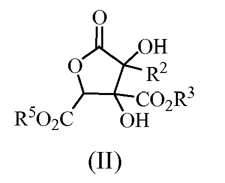 wherein
R², R³ and R⁵ are as defined above;
its stereoisomers, or mixtures thereof;
(ii) hydrolyzing said compound of formula (II) in the presence of an alkali or alkaline earth metal hydroxide, or of an alkali or alkaline earth metal carbonate; and
(iii) acidifying the reaction medium;
wherein the R₃ and R₅ groups are labile under the basic conditions of step (ii).

19. The process according to claim 18, for preparing compounds of formula (Ia') and (Ib'), or their enantiomers 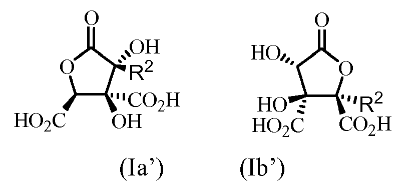 wherein
R² is as defined in claim 18;
the compound of formula (III) is a compound of formula (IIIa), or its enantiomers 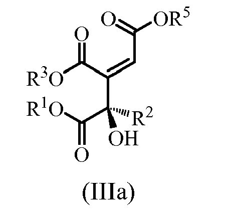 wherein R¹, R², R³ and R⁵ are as defined in claim 18; and
the compound of formula (II) is a compound of formula (IIa), or its enantiomers 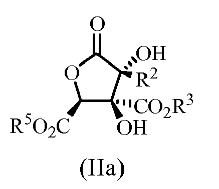 wherein R², R³ and R⁵ are as defined in the claim 18.

20. A process for preparing a compound of formula (Ia) 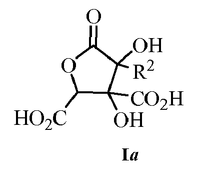 wherein
R² is a C₁₀-C₁₅ alkyl group;
its stereoisomers, or mixtures thereof,
which comprises
(i) dihydroxylating the double bond of a compound of formula (III) 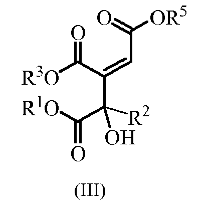 wherein
R¹ is selected from a substituted or unsubstituted C₁-C₂₀ alkyl group;
R² is as defined above; and
R³ and R⁵ are independently selected from a substituted or unsubstituted C₁-C₂₀ alkyl group;
to obtain a compound of formula (II) 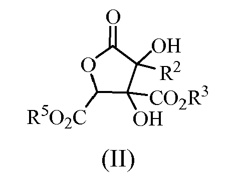 wherein
R², R³ and R⁵ are as defined above;
its stereoisomers, or mixtures thereof; and
(ii) transforming under non-basic conditions the carboxy ester groups of the lactone ring of the compound of formula (II) to obtain the corresponding carboxy acid groups.

21. A compound of formula (II) 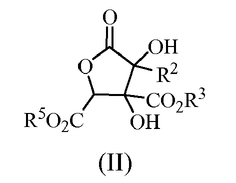 wherein
R² is selected from a C₁₀-C₁₅ s alkyl group;
R³ and R⁵ are independently selected from a substituted or unsubstituted C₁-C₂₀ alkyl group;
its stereoisomers, especially enantiomers, or mixtures thereof;
with the proviso that the compound of formula (II) is not

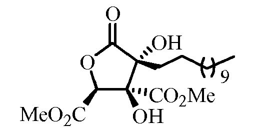

22. Use of a compound of formula (II) as defined in claim 21, for the synthesis of compounds of formula (Ia) and/or (Ib), their stereoisomers, or mixtures thereof, as well as mixtures of compounds of formula (Ia) and (Ib) or mixtures of their stereoisomers.

23. Use of at least one compound of formula (III), (V), (VI), (VII), (X), (XI) and (XIII) as defined in the previous claims for the synthesis of compounds of formula (Ia) and/or (Ib), their stereoisomers, or mixtures thereof, as well as mixtures of compounds of formula (Ia) and (Ib) or mixtures of their stereoisomers.
